# EUROPEAN PATENT SPECIFICATION

(11) **EP 0 413 450 B1**
(45) Date of publication and mention of the grant of the patent: **11.10.1995**
(21) Application number: 90308141.2
(22) Date of filing: 25.07.1990
(51) Int. Cl.: A61F 2/60

(54) **Artificial limb component**
Teil für künstliches Glied
Elément d'un membre artificiel

(30) Priority: 15.08.1989 GB 8918580
(43) Date of publication of application: 20.02.1991
(73) Proprietor: CHAS. A BLATCHFORD & SONS LIMITED, Basingstoke, Hampshire RG22 4AH (GB)
(72) Inventor: Harris, Graham James, Basingstoke, Hampshire RG22 4AH (GB)
(74) Representative: Blatchford, William Michael

(56) References cited:
- DE-B- 2 343 585
- GB-A- 1 550 658
- US-A- 2 594 945
- US-A- 3 956 775
- US-A- 3 982 280
- US-A- 4 038 705
- US-A- 4 360 931

## Description

This invention relates to an artificial limb component, and in particular to a connector for a lower limb prosthesis which allows rotation of an artificial foot with respect to a shin component about the longitudinal axis of the shin. The invention also includes a lower limb prosthesis including such a connector.

Torque absorbers in lower limb prostheses commonly include a rubber coupling element attached to the distal end of the shin component so that the foot is resiliently biased towards a rest position and is rotatable about the shin axis typically up to a maximum rotation of plus or minus 30°. Such a device is disclosed in DE-B-2343585. These known arrangements suffer from the disadvantage that the connector, in order to withstand the loads imposed upon it due to the weight of the patient and the need to achieve adequate stiffness, is constituted by a relatively heavy and bulky assembly at or near the end of the shin. In addition, it can sometimes be difficult to maintain accurately a predetermined rest position of the foot with respect to the shin due to creep in the rubber material and the lack of stiffness inherent in the material close to the rest position.

It is an object of this invention to provide an improved torque absorber.

According to a first aspect of this invention, there is provided a connector for a lower limb prosthesis for allowing rotation of the foot with respect to the shin about an axis extending longitudinally of the prosthesis, the connector comprising: a first component for mounting the shin, a second component for mounting the foot or an element connected to the foot, the first and second components having bearing surfaces allowing rotation of one component relative to the other about a rotation axis and arranged to transmit loads between the components, and means coupling the first and second components for resiliently resisting the said rotation, characterised in that the coupling means include an internal axial resilient elongate torsion element having a first end part secured to the first component, a central part, and a second end part secured to the second component, whereby the degree of rotation of the first component with respect to the second is determined by the stiffness of the torsion element and the applied rotational load, and in that the bearing surfaces are located externally of the coupling means, the coupling means and the first and second components being constructed and assembled such that when the connector is coupled to a shin component of the prosthesis the torsion element extends internally of a distal end part of the shin component or internally of an element connecting the said distal end part to the foot.

According to a second aspect of this invention, there is provided a lower limb prosthesis having a shin component, a foot, and a connector for allowing rotation of the foot with respect to the shin component about an axis extending longitudinally of the prosthesis, the connector comprising bearing means with bearing surfaces associated with the shin component and the foot or an element connected to the foot allowing the said rotation and arranged to transmit loads applied by the patient, and means coupling the shin component and the foot or the said element for resiliently resisting the said rotation, characterised in that the coupling means include an internal axial resilient elongate torsion element having a first end part secured to the shin component, a central part, and a second end part connected to the foot or the said element connected to the foot, whereby the degree of the said rotation is determined by the stiffness of the torsion element and the applied rotational load, and in that the bearing surfaces are located externally of the coupling means, the torsion element being mounted internally of a distal end part of the shin component or internally of an element connecting the said distal end part to the foot.

In a preferred embodiment of the invention the upper mounting portion is a sleeve shaped to be mounted internally in the distal end part of a shin tube, and the lower mounting portion is a housing for clamping to a foot and/or ankle assembly, the housing having a cylindrical bearing surface which engages a corresponding internal bearing surface in the sleeve, whereby the housing is rotatable with respect to the sleeve about the longitudinal axis, the torsion element defining the extent of the rotation according to the torque applied to the shin component. Typically, the stiffness of a torsion element is in the range of from 0.25 to 0.90 newton-metres per degree of rotation.

Preferably, stop means are provided to limit the rotation of the housing with respect of the sleeve to predetermined maximum angles with respect of the rest position, primarily to avoid over-stressing the torsion element. The angular limits may be in the range plus and minus 30° to 80° (preferably about 40°) from the rest position.

The torsion element may be an elongate bar or tube made of thermoplastics material such as polyamide or polyacetal. It is also possible to use a reinforced plastics material such a glass fibre reinforced epoxy resin. In some embodiments of the invention the torsion element is replaceable, being detachable from the upper and lower mounting portions. In particular, a releasable fastener may be provided on the distal end part of the torsion element accessible at or adjacent to the distal end of part of the housing extending outside the sleeve, the fastener retaining the housing in the sleeve. When the fastener, which may be a screws and/or a spring clip (for a example a circlip) are released, the housing may be withdrawn axially from the sleeve, exposing a flange formed on the proximal end part of the torsion element and screws securing the flange to an inwardly extending part of the sleeve spaced proximally from the open end of the sleeve. Removing the flange screws allows removal and replacement of the torsion element with, for example, an element of a different width or diameter having a different stiffness.

Alternatively, in the case of the torsion element being a torsion tube, replaceability may be achieved by retaining the tube in the sleeve and the housing by means of a bolt passing through the tube and threaded in the sleeve or a component associated with the sleeve, the head of the bolt retaining a tube mounting plate in the housing.

It is to be understood that the torsion element may be a part only of a generally axially extending elongate member which extends beyond one or both of the end parts of the element.

Instead of, or in conjunction with, interchangeability of torsion elements, adjustability can be built into the connector by arranging for the effective length of the torsion element to be adjustable. This may be performed by arranging for the element to have a non-circular external cross-section over at least part of its length, and providing an adjuster element which is mounted with respect to the housing so as to be adjustable in position lengthwise of the torsion element and to engage the non-circular portion of the external surface of the element at different positions. The adjuster element is preferably prevented from rotating with respect to the housing, by virtue of its shape and by being trapped between the surface of the torsion element and an internal surface of the housing. Lengthwise adjustment of the adjuster element with respect to the housing is preferably performed by means of a threaded element such as a threaded bolt passing through a threaded bore in the adjuster element.

The torsion element may be secured with respect to the sleeve and with respect to the housing either by virtue of having a non-circular section at each of its ends matching respective sockets in the sleeve and housing (or components associated with the sleeve and housing), or by means of pins located in grooves in the ends of the torsion element.

The lower mounting portion of the connector which is the housing for the distal end part of the torsion element in the preferred embodiment, advantageously has a section projecting from the shin tube and having the same external diameter as the shin tube so as to be received in the same ankle component clamp as may be used to clamp the shin tube without the connector.

The invention also includes a method of making a lower limb prosthesis as defined above, including the step of selecting a torsion element from a range of similar torsion elements having different stiffnesses by virtue, for example, of their having different widths or diameters.

The invention will now be described by way of example with reference to the drawings in which:-
- Figure 1: is a longitudinal section of a first connector in accordance with the invention shown mounted in a distal end portion of a shin tube;
- Figure 2: is a cross-section of the connector on the line X-X in Figure 1;
- Figure 3: is a longitudinal section of a second connector in accordance with the invention shown mounted in the distal end portion of the shin tube;
- Figure 4: is an end view of the distal end of the connector as shown by the arrow A in Figure 3;
- Figure 5: is a side elevation of a torsion tube used in the connector of Figure 3;
- Figure 6: is an end elevation of the torsion tube;
- Figure 7: is an enlarged cross-section of the torsion tube on the line X-X in Figure 5;
- Figure 8: is an end elevation of an adjuster element of the connector of Figure 3;
- Figure 9: is a sectioned side view of a housing of the connector of Figure 3;
- Figure 10: is a distal end elevation of the housing of Figure 9;
- Figure 11: is a proximal end elevation of the housing of Figure 9;
- Figure 12: is a longitudinal section of a third connector in accordance with the invention shown mounted in a distal end portion of the shin tube;
- Figure 13: is a side elevation of the torsion element of the third rotator connector;
- Figure 14: is a distal end elevation of the torsion element of Figure 13;
- Figure 15: is a proximal end elevation of a sleeve shown in Figure 12;
- Figure 16: is a flange washer associated with the torsion element of Figure 13;
- Figure 17: is a cross-section through part of the housing of the connector on the line X-X in Figure 12;
- Figure 18: is a longitudinal cross-section through the same housing; and
- Figure 19: is a proximal end elevation of the housing.

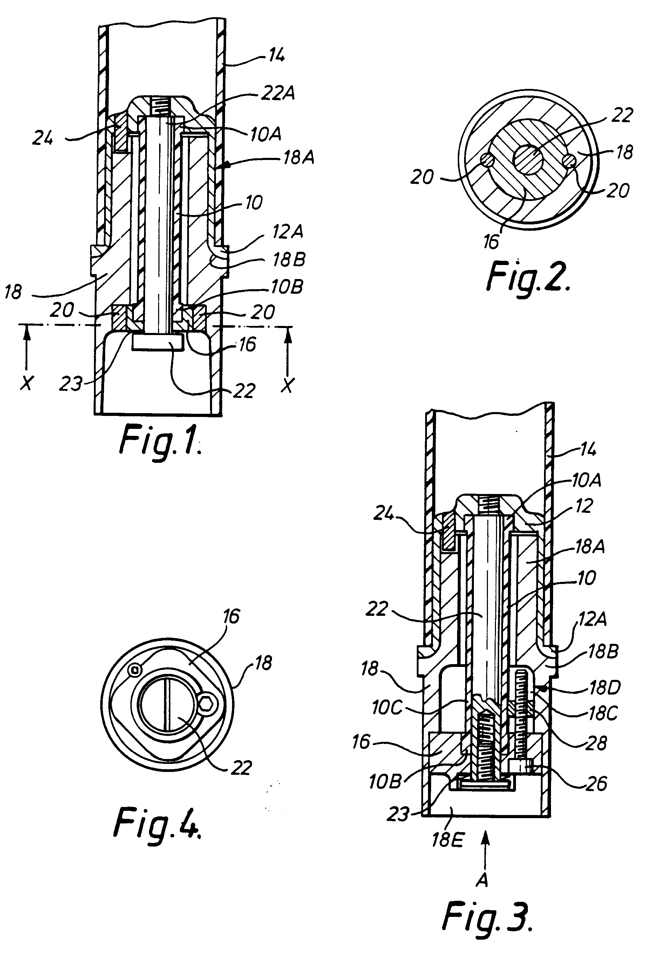

Referring to Figures 1 and 2 of the drawings, a connector in accordance with the invention has a torsion element in the form of a tube 10 with one end 10A fixed in a sleeve 12 housed in a distal end portion of a shin tube 14 of an endoskeletal lower limb prosthesis. The other end 10B of the torsion tube 10 is fixed in an anchor plate 16 which is, in turn, fixed in a housing 18 extending distally beyond the end of the shin tube 14 and free to rotate within the sleeve 12. To allow such rotation, the housing 18 has a cylindrical bearing surface portion 18A having a diameter corresponding to the internal diameter of the sleeve 12. This bearing surface portion 18A is treated with lubricant which may be a dry lubricant incorporating, for example, polytetrafluoroethylene. Longitudinal compressive forces applied to the sleeve 12 are transmitted to the housing 18 by means of an external shoulder 18B on the housing which cooperates with a rim portion 12A of the sleeve.

It will be noted that the external diameter of the projecting portion of the housing having the bearing surface portion 18A is identical to the external diameter of the shin tube 14. This provides for compatibility between lower limb prostheses incorporating a connector in accordance with the invention with prostheses having no such component, the same connecting clamp on a foot/ankle assembly (not shown) being suitable for both.

Rotation of the housing with respect to the sleeve is controlled by the torsion tube 10. In the example shown in the drawings, the end portions 10A and 10B of the torsion tube 10 are of non-circular, preferably square, cross-section to prevent rotation of the tube with respect to correspondingly shaped sockets in an end cap portion of the sleeve 12 and the anchor plate 16. Alternatively, the end portions 10A and 10B of the torsion tube may be circular and located against rotation by means of pins housed in oppositely located grooves in the tube end portions 10A and 10B and the walls of the sockets in the sleeve 12 and the anchor plate 16 respectively.

The torsion tube 10 in this embodiment is moulded from a polyacetal material such as that sold under the trade mark Delrin. The thickness and diameter of the tube are such that the rotational stiffness of the connector is in the range of from 0.25 to 0.90 newton metres per degree of rotation.

The anchor plate 16 is prevented from rotating in the housing 18 by a pair of pins 20 mounted with their axes parallel to the longitudinal axis of the connector and in part-cylindrical grooves cut in the periphery of the anchor plate 16 in registry with corresponding grooves in a recess of the housing 18. A central hole is provided in the anchor plate 16 to receive a bolt 22 which passes through the torsion tube 10 and is threaded in the end cap of the sleeve 12. A spring washer 23 is placed between the head of the bolt 22 and the anchor plate 16, while, to avoid compression of the bearing surfaces of the housing 18 and the sleeve 12, the bolt 22 has a shoulder 22A which engages the distal surface of the sleeve 12.

Typically, the shin tube is manufactured from a fibre-reinforced plastics material, while the sleeve and the housing are machined aluminium alloy components.

The maximum rotation allowed by the connector shown in Figures 1 and 2 is limited by a limiter pin 24 housed off-centre in the end cap of the sleeve 12 and projecting into a recess formed in the proximal end of the housing 18. According to the angles subtended by the ends of the recess at the longitudinal axis of the connector, the rotation is limited to predetermined angles such as plus or minus 30° with respect to the rest position defined by the position of the housing 18 with respect to the sleeve 12 when no torque is applied.

Other materials may be used for the torque tube 10, especially polyamide (Nylon), or fibre-reinforced plastics, depending upon the required rotational stiffness.

It will be appreciated that the torsion tube 10 is easily replaced by unscrewing the bolt 22 from the end cap of the sleeve 12, and extracting the anchor plate 16.

Referring to Figures 3 and 4, a second embodiment of connector in accordance with the invention will now be described. In this embodiment, the rotational stiffness of the connector is adjustable by altering the effective length of the torsion tube 10.

Insofar as this second embodiment possesses a torsion tube 10, a sleeve 12, a housing 18, and an anchor plate 16, it is similar to the embodiment shown in Figures 1 and 2. As before, the housing 18 has a bearing surface 18A which engages the internal surface of the sleeve 12, and longitudinal loads are transmitted by a shoulder 18B of the housing and the rim 12A of the sleeve 12.

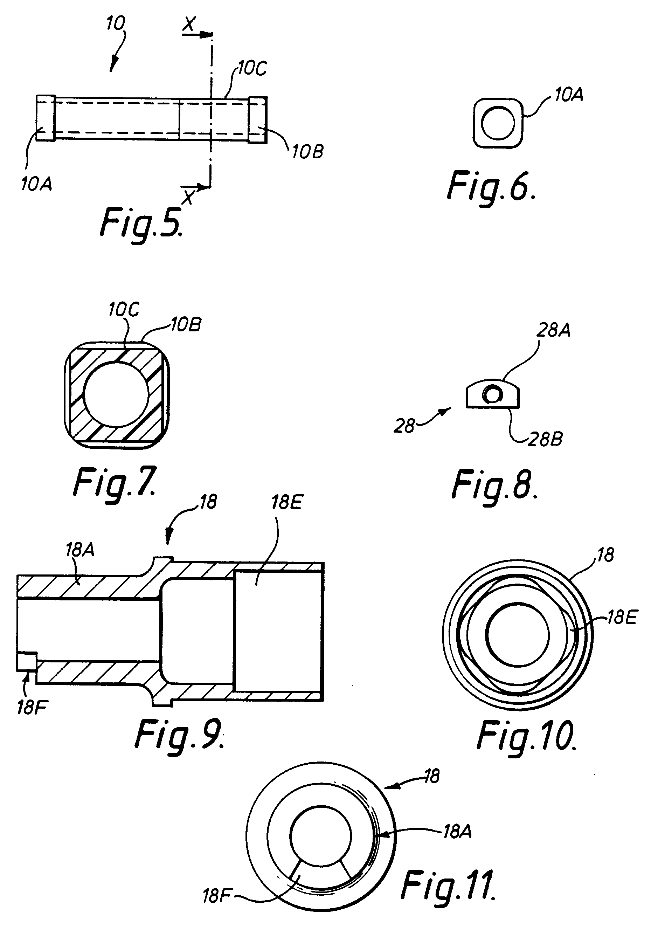

However, in this embodiment, the torsion tube 10, over a portion 10C of its length (see Figure 5) has a squared external cross-section, as shown in Figure 7. Mounted in a bore in the anchor plate 16 is an adjusting screw 26 extending to one side of and parallel to the torsion tube 10 in registry with the squared section 10C. A chamber 18C is provided in the housing to accommodate the screw 26 and has a wall 18D shaped to receive a non-rotatable nut 28 which is threaded on the adjusting screw 26. The nut is shown in Figure 8. As will be seen, one surface 28A of the nut 28 is part-cylindrical to mate with the cylindrical wall 18D of the housing chamber 18C (see Figure 9), while a planar face 28B opposite the surface 28A engages one of the flats of the squared torsion tube portion 10C. Being located on the adjusting screw 26, the nut 28 prevents rotation of the torsion tube 10 with respect to the housing 18 at all points distally of the nut 28. By rotating the adjusting screw 26, the position of the nut 28 can be altered, thereby altering the effective length of the torsion tube 10 in order to adjust the rotational stiffness of the connector.

In this second embodiment, the anchor plate 16 is of a rounded square cross-section to fit in a corresponding squared recess 18E of the housing 18 (see Figures 4, 9, and 10). As before, the ends 10A and 10B of the torsion tube 10 are squared as shown in Figure 6 to prevent rotation with respect to sleeve 12 and the housing 18.

Another feature visible in the drawings is the recess, numbered 18F in Figures 9 and 11, of the housing 18 for the stop pin 24.

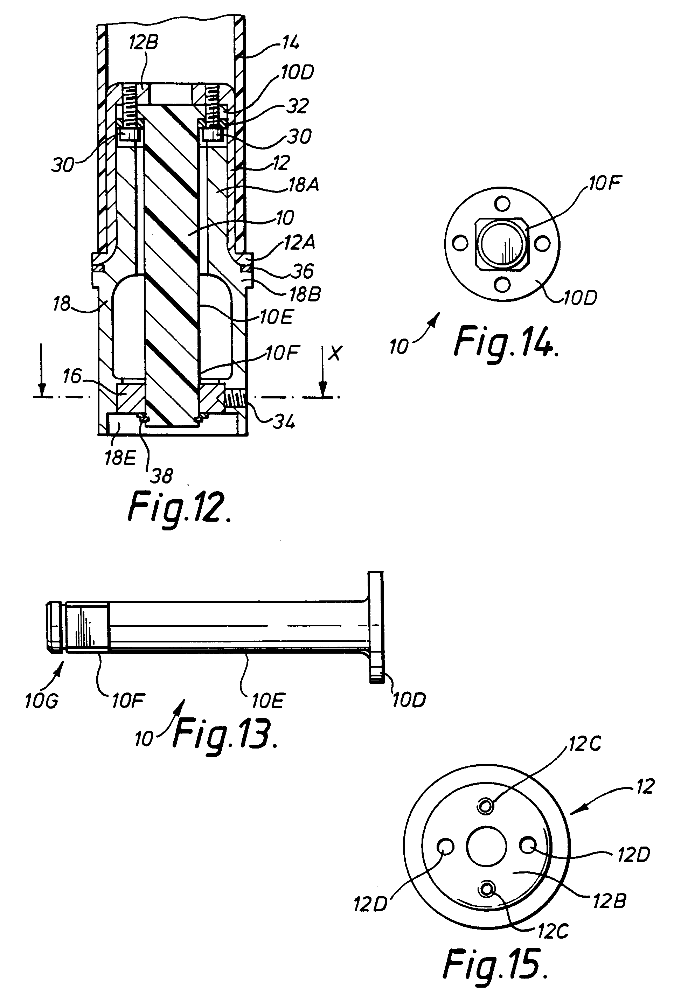

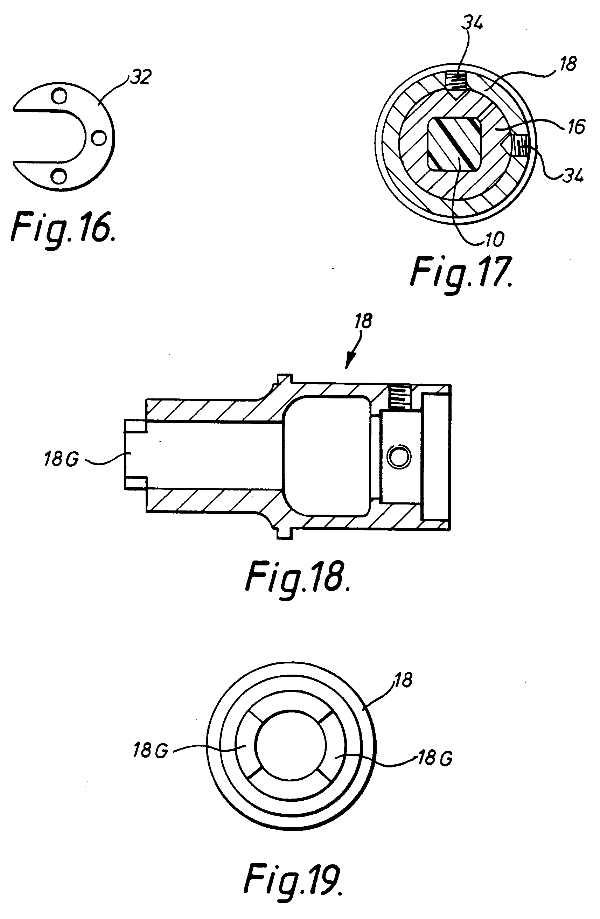

Referring now to Figures 12, 13, and 14, a third embodiment of the invention makes use of a torsion element 10 in the form of a polyamide (Nylon) bar 10 having at one end a flange 10D, a cylindrical central portion 10E of a predetermined diameter, and an opposite end part of 10F of square cross-section. Four holes are formed in the flange 10D to accept two screws 30 which are threaded in a generally radially inwardly extending part of end wall 12B of the sleeve (see Figure 12) and two dowels (not shown) which are an interference fit in end wall 12B. The arrangement of threaded and unthreaded holes 12C and 12D respectively in the end wall 12B of the sleeve 12 is shown in Figure 15. The heads of the screws 30 bear against a washer 32 (shown in Figure 16). The length of the dowels mounted in the holes 12D of the sleeve 12 is arranged such that their lower ends are flush with the lower surface of the washer 32. The heads of the screws 30 project distally of the washer 32.

The housing 18 is similar to the housings of the first and second embodiments described above with reference to Figures 1 to 11. However, an anchor element 16 received in the distal end of the housing takes the form of a split ring with a square central aperture to match the square cross-section of the distal end part of the torsion element 10, as shown in Figure 16. Two grub screws 34 threaded in the side of the housing are used to compress the anchor ring 16 for gripping the torsion element and to prevent rotation of the anchor ring in the housing 18.

As before, the housing 18 is rotatably received within the sleeve 12 which is itself bonded within the shin tube 14. Again, the housing has an external shoulder portion 18B which bears against an end lip 12A at the open end of the sleeve 12 for transmitting longitudinally applied loads. In this case, a plastics bearing washer 36 is inserted between the shoulder 18D and the lip 12A to reduce wear (see Figure 12).

It will be appreciated that the torsion bar 10 is prevented from rotating at one end with respect of the sleeve 12 and at the other end with respect to the housing 18 so that the degree of rotation of the housing in the sleeve 12 about the longitudinal axis is determined by the rotational load applied and by the stiffness of the torsion bar.

In accordance with one aspect of the invention, a range of torsion bars may be provided, having different diameters over the lengths of their central sections 10E, or being made of different materials. These may be differently coloured for identification.

In this embodiment the housing 18 is retained in the sleeve 12 by means of a fastener associated with the distal end of the torsion bar 10 which projects through the anchor element 16. Adjacent the distal end of the bar 10, there is a groove 10G (see Figure 13) for receiving a fastener 38 in the form of an annular clip (circlip) 38. This clip is accessible from the end of the housing 18 when the foot and/or ankle assembly is removed. Releasing the clip 38 and grub screws 34 allows the housing 18 to be slidably withdrawn from the sleeve 12, thereby exposing the heads of the screws 30. Removal of the screws 30 allows the torsion bar to be withdrawn and replaced.

As in the previous embodiments, stop means are provided for limiting the degree of rotation of the housing 18 with respect of the sleeve 12. In this case, the proximal end of the housing has two projections 18C on opposite sides of the centre line. The circumferential extent of each projection is such that two recesses are formed each subtending an angle of approximately 120° at the central axis. As will be seen from a comparison of Figures 18 and 19 with Figure 12, rotation of the housing 18 is limited by the abutment of the edges of the projections 18C against the projecting heads of the screws 30 beneath the washer 32 and flange 10D.

The invention is of general use to improve the comfort of the limb for the patient, in particular by reducing torsional shocks on the stump socket, but is also especially useful for golfing patients who require rotational flexibility to achieve a satisfactory swing.

## Claims

1. A connector for a lower limb prosthesis for allowing rotation of the foot with respect to the shin about an axis extending longitudinally of the prosthesis, the connector comprising:
a first component (12) for mounting the shin,
a second component (18) for mounting the foot or an element connected to the foot,
the first and second components having bearing surfaces allowing rotation of one component relative to the other about a rotation axis and arranged to transmit loads between the components, and
means coupling the first and second components for resiliently resisting the said rotation, the coupling means including an internal axial resilient torsion element (10) having a first axial end part (10A) secured to the first component (12), a central part, and a second axial end part (10B) secured to the second component (18), whereby the degree of rotation of the first component with respect to the second is determined by the stiffness of the torsion element (10) and the applied rotational load, the coupling means and the first and second components being constructed and assembled such that when the connector is coupled to a shin component (14) of the prosthesis the torsion element (10) extends internally of a distal end part of the shin component or internally of an element connecting the said distal end part to the foot, characterised in that the torsion element (10) is elongate and in that the bearing surfaces are located radially externally of the torsion element (10).

2. A connector according to claim 1, characterised in that the torsion element (10) has a stiffness in the range of from 0.25 to 0.90 newton-metres per degree of rotation.

3. A connector according to claim 1 or 2, characterised in that the torsion element (10) is a bar or tube made of a thermoplastics material.

4. A connector according to claim 3, characterised in that the torsion element (10) is made of polyacetal, polyamide or a fibre-reinforced plastics material.

5. A connector according to any preceding claim, characterised in that the effective length of the torsion element (10) is adjustable.

6. A connector according to any preceding claim, characterised in that at least one of the end parts of the torsion element (10) is non-circular in cross-section and is located in a corresponding non-circular socket in the respective mounting portion (12, 18).

7. A connector according to any preceding claim, characterised in that the second component (18) has an external portion (18B) which bears against the first component (12) for transmitting longitudinally applied loads.

8. A connector according to any preceding claim, characterised in that the first component (12) is a sleeve for mounting inside the distal end part of a shin component (14), the sleeve (12) having an open end and an inwardly extending part located remotely from the open end and receiving one end part of the torsion element (10), and in that the second component (18) is a housing for the other end part of the torsion element (10) and is rotatably received within the sleeve (12), part of the housing (18) extending longitudinally outside the sleeve (12) for connection to a foot and/or ankle assembly, the dorsi element (10) defining the extent of the rotation according to the torque applied to the connector.

9. A connector according to claim 8, characterised by a releasable fastener (38) associated with the said other end part of the torsion element (10) and accessible at or adjacent an end of the said part of the housing (18) extending outside the sleeve (12), the fastener (38) retaining the housing (18) in the sleeve (12), and further including releasable means (30) for securing the said one end part of the torsion element (10) to the inwardly extending part of the sleeve (12).

10. A connector according to claim 9, characterised in that the said one end part of the torsion element (10) has a flange (10D) and the releasable securing means (30) comprise at least one screw accessible inside the sleeve (12) when the housing (18) is withdrawn from the sleeve (12).

11. A connector according to any of claims 8 to 10, characterised in that the housing (18) has an external shoulder (18B)which bears against an open end edge of the sleeve (12).

12. A lower limb prosthesis having a shin component (14), a foot, and a connector for allowing rotation of the foot with respect to the shin component about an axis extending longitudinally of the prosthesis, the connector comprising bearing means with bearing surfaces associated with the shin component (14) and the foot or an element connected to the foot allowing the said rotation and arranged to transmit loads applied by the patient, and means coupling the shin component (14) and the foot or the said element for resiliently resisting the said rotation, the coupling means including an internal axial resilient torsion element (10) having a first axial end part (10A) secured to the shin component, a central part, and a second axial end part (10B) connected to the foot or the said element connected to the foot, whereby the degree of the said rotation is determined by the stiffness of the torsion element (10) and the applied rotational load, the torsion element (10) being mounted internally of a distal end part of the shin component (14) or internally of an element connecting the said distal end part to the foot, characterised in that the torsion element (10) is elongate and in that the bearing surfaces are located radially externally of the torsion element (10).

13. A prosthesis according to claim 12, characterised in that the connector comprises a connection between the distal end part of the shin component (14) and an ankle component.

14. A prothesis according to claim 12 or claim 13, characterised in that the first and second end parts of the torsion element (10) are respectively secured in upper and lower mounting portions (12, 18) which are rotatable with respect to each other, and in that the lower mounting portion has an external portion (18B) which bears against the upper mounting portion for transmitting longitudinally applied loads.

15. A prosthesis according to any of claims 12 to 14, characterised in that the shin component (14) is a shin tube and in that an upper end part of the torsion element (10) is secured in a sleeve (12) mounted internally of the shin tube (14), and a lower end part of the torsion element (10) is secured in a housing (18) clamped to a foot and/or ankle assembly, the housing (18) having a cylindrical bearing surface (18A) which engages a corresponding internal bearing surface in the sleeve (12), whereby the housing (18) is rotatable with respect to the sleeve (12) about the shin axis, the torsion element (10) defining the extent of rotation according to the torque applied to the shin component.

16. A prothesis according to any of claims 12 to 15, characterised in that the torsion element (10) is made of polyacetal, polyamide or a fibre-reinforced plastics material.

17. A prosthesis according to any of claims 12 to 16, characterised in that the effective length of the torsion element (10) is adjustable.

18. A prosthesis according to any of claims 12 to 17, characterised in that the shin component (14) is a shin tube and one end part of the torsion element (10) is secured to an inwardly extending element inside the shin tube (14) at a location spaced from the distal end of the shin tube (14), and wherein the other end part of the torsion element (10) is secured to a housing (18) which is rotatably received within the distal end part of the shin tube (14), part of the housing (18) extending longitudinally outside the shin tube (14) and being connected to the foot.

19. A prosthesis according to claim 18, characterised by a releasable fastener (38) associated with the said other end of the torsion element (10) and accessible at or adjacent an end of the said part of the housing (18) outside the shin tube (14), the fastener (38) retaining the housing (18) in the sleeve (12), and further including releasable means (30) for securing the said one end part of the torsion element (10) to the inwardly extending part of the sleeve (12).

20. A lower limb prosthesis in which the foot is rotatable relative to the shin about an axis extending longitudinally of the prosthesis, characterised by a connecting component as defined in any of claims 1 to 9.

21. A method of making a lower limb prosthesis as defined in any of claims 12 to 20, characterised by the step of selecting a torsion element (10) from a range of similar torsion elements having different stiffnesses.

22. A method according to claim 21, characterised in that the torsion elements of the said range have different widths, the greater the width of an element the greater is its rotational stiffness about its longitudinal axis.

## Patentansprüche

1. Verbinder für eine Unterschenkelprothese zum Ermöglichen einer Drehung des Fußes gegenüber dem Schienbein um eine Achse, die sich in Längsrichtung der Prothese erstreckt, wobei der Verbinder umfaßt:
ein erstes Bauteil (12) zum Befestigen des Schienbeins,
ein zweites Bauteil (18) zum Befestigen des Fußes oder eines mit dem Fuß verbundenen Elements,
wobei die ersten und zweiten Bauteile Lagerflächen aufweisen, die eine Drehung des einen Bauteils gegenüber dem anderen um eine Drehachse erlauben und die zum Übertragen von Belastungen zwischen den Bauteilenangeordnet sind, und
Mittel, welche das erste und zweite Bauteil in der Weise verbinden, daß sie der besagten Drehung elastisch widerstehen,
wobei die Verbindungsmittel ein inneres axial elastisches Drehelement (10) umfassen, welches einen ersten axialen Endbereich (10A) aufweist, der mit dem ersten Bauteil (12) verbunden ist, ferner einen zentralen Bereich, und einen zweiten axialen Endbereich (10B), welcher mit dem zweiten Bauteil (18) verbunden ist, wodurch der Grad der Drehung des ersten Bauteils gegenüber dem zweiten bestimmt wird durch die Steifigkeit des Torsionselements (10) und die aufgebrachte Drehlast, wobei die Verbindungsmittel und die ersten und zweiten Bauteile derart aufgebaut und zusammengesetzt sind, daß, wenn der Verbinder mit einem Schienbeinbauteil (14) der Prothese verbunden ist, das Torsionselement (10) sich innerhalb eines beabstandeten Endbereichs des Schienbeinbauteils oder innerhalb eines Elements erstreckt, welches den genannten beabstandeten Endbereich mit dem Fuß verbindet,
dadurch gekennzeichnet,
daß das Torsionselement (10) länglich ausgebildet ist und daß die Lagerflächen radial an der Außenseite des Torsionselements (10) angeordnet sind.

2. Verbinder nach Anspruch 1,
dadurch gekennzeichnet,
daß das Torsionselement (10) eine Steifigkeit im Bereich von 0,25 bis 0,90 Newton-Meter pro Grad Drehung aufweist.

3. Verbinder nach Anspruch 1 oder 2,
dadurch gekennzeichnet,
daß das Torsionselement (10) eine Stange oder ein Rohr aus thermoplastischem Material ist.

4. Verbinder nach Anspruch 3,
dadurch gekennzeichnet,
daß das Torsionselement (10) aus Polyacetal, Polyamid oder faserverstärktem Kunststoffmateril besteht.

5. Verbinder nach einem der vorhergehenden Ansprüche,
dadurch gekennzeichnet,
daß die wirksame Länge des Torsionselements (10) einstellbar ist.

6. Verbinder nach einem der vorhergehenden Ansprüche,
dadurch gekennzeichnet,
daß mindestens einer der Endbereiche des Torsionselements (10) in seinem Querschnitt unrund ist und innerhalb einer korrespondierenden unrunden Fassung in dem entsprechenden Montagebereich (12, 18) angeordnet ist.

7. Verbinder nach einem der vorhergehenden Ansprüche,
dadurch gekennzeichnet,
daß das zweite Bauteil (18) einen äußeren Bereich (18B) hat, der für ein Übertragen von in Längsrichtung auftretenden Belastungen an dem ersten Bauteil (12) anliegt.

8. Verbinder nach einem der vorhergehenden Ansprüche,
dadurch gekennzeichnet,
daß das erste Bauteil (12) eine Hülse zur Montage innerhalb des beabstandeten Endbereichs des Schienbeinbauteils (14) ist, wobei die Hülse (12) ein offenes Ende und einen nach innen sich erstreckenden Bereich aufweist, der von dem offenen Ende entfernt liegt und einen Endbereich des Torsionselements (10) aufnimmt, und daß das zweite Bauteil (18) ein Gehäuse für den anderen Endbereich des Torsionselements (10) ist und drehbar innerhalb der Hülse (12) aufgenommen ist, wobei ein Teil des Gehäuses (18) sich in Längsrichtung außerhalb der Hülse (18) für eine Verbindung mit einem Fuß- oder Enkelaufbau erstreckt, wobei das Torsionselement (10) das Ausmaß der Drehung entsprechend dem auf den Verbinder ausgeübten Drehmoment bestimmt.

9. Verbinder nach Anspruch 8,
gekennzeichnet durch
ein lösbares Befestigungsteil (38), das mit dem genannten anderen Endbereich des Torsionselements (10) verbunden ist und zugänglich am oder benachbart zu einem Ende des genannten Teils des Gehäuses ist, das sich außerhalb der Hülse (12) erstreckt, wobei das Befestigungsteil (38) das Gehäuse (18) in der Hülse (12) festhält, ferner mit lösbaren Mitteln (30) zum Sichern des genannten Endbereichs des Torsionselements (10) gegenüber dem einwärts sich erstreckenden Teil der Hülse (12).

10. Verbinder nach Anspruch 9,
dadurch gekennzeichnet,
daß der genannte Endbereich des Torsionselements (10) einen Flansch (10D) hat und die entfernbaren Sicherungsmittel (30) mindestens eine Schraube umfassen, die innerhalb der Hülse (12) zugänglich ist, wenn das Gehäuse (18) aus der Hülse (12) zurückgezogen wird.

11. Verbinder nach einem der Ansprüche 8 bis 10,
dadurch gekennzeichnet,
daß das Gehäuse (18) eine äußere Schulter (18B) aufweist, die an einer offenen Endkante der Hülse (12) anliegt.

12. Schenkelprothese mit einem Schienbeinbauteil (14), einem Fuß und einem Verbinder, der eine Drehung des Fußes gegenüber dem Schienbeinbauteil um eine Achse erlaubt, die sich in Längsrichtung der Prothese erstreckt, wobei der Verbinder Lagermittel mit Lagerflächen umfaßt, die mit dem Schienbeinbauteil (14) und dem Fuß oder einem mit dem Fuß verbundenen Bauteil verbunden sind, welche die genannte Drehung erlauben und angeordnet sind für ein Übertragen von durch den Patienten aufgebrachten Belastungen, und Mittel, welche das Schienbeinbauteil (14) und den Fuß oder das genannte Bauteil mit einem elastischen Widerstand gegen die genannte Drehung verbinden, wobei die Verbindungsmittel ein inneres axiales, elastisches Torsionselement (10) aufweisen, welches einen ersten Endbereich (10A) aufweist, der mit dem Schienbeinbauteil verbunden ist, ferner einen zentralen Bereich, und einen zweiten axialen Endbreich (10B), der mit dem Fuß oder dem genannten, mit dem Fuß verbundenen Bauteil verbunden ist, wodurch der Grad der Drehung durch die Steifigkeit des Torsionselements (10) und die aufgebrachte Drehlast bestimmt wird, wobei das Torsionselement (10) innerhalb eines beabstandeten Endbereichs des Schienbeinbauteils (14) oder innerhalb eines Bauteils montiert ist, das den genannten beabstandeten Endbereich mit dem Fuß verbindet,
dadurch gekennzeichnet,
daß das Torsionselement (10) länglich ist und daß die Lagerflächen radial außerhalb des Torsionselements (10) angeordnet sind.

13. Prothese nach Anspruch 12,
dadurch gekennzeichnet,
daß der Verbinder eine Verbindung zwischen dem beabstandeten Endbereich des Schienbeinbauteils (14) und einem Fußknöchelbauteil umfaßt.

14. Prothese nach Anspruch 12 oder 13,
dadurch gekennzeichnet,
daß der erste und der zweite Endbereich des Torsionselements (10) jeweils in oberen und unteren Montagebereichen (12, 18) gesichert sind, welche gegeneinander drehbar sind, und daß der untere Montagebereich einen äußeren Bereich (18B) aufweist, der an dem oberen Montagebereich für ein Übertragen von in Längsrichtung aufgebrachten Belastungen anliegt.

15. Prothese nach einem der Ansprüche 12 bis 14,
dadurch gekennzeichnet,
daß das Schienbeinbauteil (14) ein Schienbeinrohr ist und daß ein oberer Endbereich des Torsionselements (10) in einer Hülse (12) gesichert ist, die innerhalb des Schienbeinrohrs (14) montiert ist, wobei ein unterer Endbereich des Torsionselements (10) in einem Gehäuse (18) gesichert ist, der an einem Fuß- oder Enkelaufbau befestigt ist, wobei das Gehäuse (18) eine zylindrische Lagerfläche (18A) hat, die im Eingriff steht mit einer inneren Lagerfläche in der Hülse (18), wodurch das Gehäuse (18) gegenüber der Hülse (12) um die Schienbeinachse drehbar ist, wobei das Torsionselement (10) das Ausmaß der Drehung entsprechend dem auf das Schienbeinbauteil ausgeübten Drehmoment bestimmt.

16. Prothese nach einem der Ansprüche 12 bis 15,
dadurch gekennzeichnet,
daß das Torsionselement (10) aus Polyacetal, Polyamid oder einem faserverstärkten Kunststoffmaterial besteht.

17. Prothese nach einem der Ansprüche 12 bis 16,
dadurch gekennzeichnet,
daß die wirksame Länge des Torsionselements (10) einstellbar ist.

18. Prothese nach einem oder Ansprüche 12 bis 17,
dadurch gekennzeichnet,
daß das Schienbeinbauteil (14) ein Schienbeinrohr ist und ein Endbereich des Torsionselements (10) an einem einwärts sich erstreckenden Bauteil innerhalb des Schienbeinrohrs (14) befestigt ist an einer Stelle, die von dem beabstandeten Ende des Schienbeinrohrs (14) entfernt liegt, und wobei der andere Endbereich des Torsionselements (10) an einem Gehäuse (18) befestigt ist, das drehbar innerhalb des beabstandeten Endbereichs des Schienbeinrohrs (14) aufgenommen ist, wobei ein Teil des Gehäuses (18) sich in Längsrichtung außerhalb des Schienbeinrohrs (14) erstreckt und mit dem Fuß verbunden ist.

19. Prothese nach Anspruch 18,
dadurch gekennzeichnet,
daß ein lösbares Befestigungsteil (38) mit dem genannten anderen Ende des Torsionselements (10) verbunden und zugänglich an oder benachbart zu einem Ende des genannten Teils des Gehäuses (18) außerhalb des Schienbeinrohrs (14) ist, wobei das Befestigungsteil (38) das Gehäuse (18) in der Hülse hält und wobei ferner lösbare Mittel (30) für ein Sichern des genannten einen Endbereichs des Torsionselements (10) gegenüber dem einwärts sich erstreckenden Teil der Hülse (12) vorgesehen sind.

20. Schenkelprothese, bei der der Fuß drehbar gegenüber dem Schienbein ist um eine Achse, die sich in Längsrichtung der Prothese erstreckt,
gekennzeichnet durch ein Verbinderbauteil, wie es in einem der Ansprüche 1 bis 9 definiert ist.

21. Verfahren zum Herstellen einer Schenkelprothese, wie sie in einem der Ansprüche 10 bis 20 definiert ist,
gekennzeichnet durch den Schritt, daß ein Torsionselement (10) aus einer Reihe von ähnlichen Torsionselementen mit unterschiedlichen Steifigkeiten ausgewählt wird.

22. Verfahren nach Anspruch 21,
dadurch gekennzeichnet,
daß die Torsionselemente der genannten Reihe unterschiedliche Durchmesser haben, wobei die Drehsteifigkeit um ihre Längsachsen um so größer ist, je größer der Durchmesser eines Elements ist.

## Revendications

1. Articulation pour prothèse de membre inférieur permettant la rotation du pied par rapport à l'axe de la jambe autour d'un axe s'étendant suivant la longueur de la prothèse, l'articulation comprenant :
un premier composant (12) pour le montage de l'axe de la jambe,
un second composant (18) pour le montage du pied ou d'un élément relié au pied,
le premier et le second composants comportant des surfaces portantes permettant la rotation d'un premier composant par rapport à l'autre autour d'un axe de rotation et disposées de façon à transmettre les charges entre les composants, et
des moyens pour coupler le premier et le second composants pour fournir une résistance élastique à l'encontre de ladite rotation, lesdits moyens de couplage comprenant un élément de torsion élastique axial interne (10) ayant une première partie d'extrémité axiale (10A) fixée au premier composant (12), une partie centrale, et une seconde partie d'extrémité axiale (10B) fixée au second composant (18), grâce à quoi le degré de rotation du premier composant par rapport au second est déterminé par la rigidité de l'élément de torsion (10) et la charge de rotation appliquée, les moyens de couplage et le premier et le second composants étant construits et assemblés de telle façon que lorsque l'articulation est couplée à un composant (14) de jambe de la prothèse l'élément de torsion (10) s'étend intérieurement à la partie d'extrémité distale du composant de jambe ou intérieurement à un élément de liaison de ladite partie d'extrémité distale au pied, ladite articulation étant caractérisée en ce que l'élément de torsion (10) est allongé et en ce que les surfaces de portée sont disposées radialement extérieurement à l'élément de torsion (10).

2. Articulation selon la revendication 1, caractérisée en ce que l'élément de torsion (10) présente une rigidité comprise dans l'intervalle de 0,25 à 0,90 newton-mètre par degré de rotation.

3. Articulation selon la revendication 1 ou la revendication 2, caractérisée en ce que l'élément de torsion (10) est une barre ou un tube constitué en un matériau thermoplastique.

4. Articulation selon la revendication 3, caractérisée en ce que l'élément de torsion (10) est fabriqué en un polyacétal, un polyamide ou un matériau plastique renforcé par des fibres.

5. Articulation selon l'une quelconque des revendications précédentes, caractérisée en ce que la longueur effective de l'élément de torsion (10) est réglable.

6. Articulation selon l'une quelconque des revendications précédentes, caractérisée en ce qu'au moins une des parties d'extrémité de l'élément de torsion (10) présente une section transversale non circulaire et est logée dans un alvéole correspondant non circulaire dans la partie de montage respective (12, 18).

7. Articulation selon l'une quelconque des revendications précédentes, caractérisée en ce que le second composant (18) présente une partie externe (18B) qui porte contre le premier composant (12) pour transmettre les charges appliquées longitudinalement.

8. Articulation selon l'une quelconque des revendications précédentes, caractérisée en ce que le premier composant (12) est un manchon pour le montage interne de la partie d'extrémité distale d'un composant (14) de jambe, le manchon (12) comportant une extrémité ouverte et une partie s'étendant vers l'intérieur disposée à distance de l'extrémité ouverte et recevant une partie d'extrémité de l'élément de torsion (10), et en ce que le second composant (18) est un logement pour l'autre partie d'extrémité de l'élément de torsion (10) et est reçu à rotation à l'intérieur du manchon (12), une partie du logement (18) s'étendant longitudinalement à l'extérieur du manchon (12) pour sa liaison à l'ensemble de cheville et/ou de pied, l'élément dorsal (10) définissant l'étendue de la rotation selon le couple appliqué à l'articulation.

9. Articulation selon la revendication 8, caractérisée par un dispositif de fixation libérable (38) associé à l'autre partie d'extrémité de l'élément de torsion (10) et accessible à ou au voisinage d'une extrémité de ladite partie du logement (18) s'étendant à l'extérieur du manchon (12), le dispositif de fixation (38) retenant le logement (18) dans le manchon (12), et comprenant en outre des moyens libérables (30) pour fixer ladite première partie d'extrémité de l'élément de torsion (10) à la partie qui s'étend vers l'intérieur du manchon (12).

10. Articulation selon la revendication 9, caractérisée en ce que ladite première partie d'extrémité de l'élément de torsion (10) présente un flasque (10D) et les moyens de fixation libérables (30) comprennent au moins une vis accessible à l'intérieur du manchon (12) lorsque le logement (18) est retiré du manchon (12).

11. Articulation selon l'une quelconque des revendications 8 à 10, caractérisée en ce que le boîtier (18) comporte un épaulement externe (18B) qui porte contre un rebord d'extrémité ouvert du manchon (12).

12. Prothèse de membre inférieur comportant un composant de jambe (14), un pied, et une articulation pour permettre la rotation du pied par rapport au composant de jambe autour d'un axe s'étendant suivant la longueur de la prothèse, l'articulation comprenant des moyens de portée avec des surfaces de portée associées au composant (14) de jambe et le pied ou un élément relié au pied permettant ladite rotation et étant disposées de façon à transmettre les charges appliquées par le patient, et des moyens assurant le couplage du composant de jambe (14) et du pied ou dudit élément résistant élastiquement à ladite rotation, les moyens de couplage comprenant un élément de torsion élastique axial interne (10) présentant une première partie d'extrémité axiale (10A) fixé au composant de jambe, une partie centrale, et une seconde partie d'extrémité axiale (10B) reliée au pied ou audit élément relié au pied, grâce à quoi le degré de ladite rotation est déterminé par la rigidité de l'élément de torsion (10) et par la charge de rotation appliquée, l'élément de torsion (10) étant monté à l'intérieur d'une partie d'extrémité distale du composant de jambe (14) ou intérieurement à un élément reliant ladite partie d'extrémité distale au pied, ladite prothèse étant caractérisée en ce que l'élément de torsion (10) est allongé et en ce que les surfaces de portée sont disposées radialement extérieurement audit élément de torsion (10).

13. Prothèse selon la revendication 12, caractérisée en ce que l'articulation comprend une liaison entre la partie d'extrémité distale du composant de jambe (14) et un composant de cheville.

14. Prothèse selon la revendication 12 ou la revendication 13, caractérisée en ce que la première et la seconde parties d'extrémité de l'élément de torsion (10) sont fixées respectivement dans les parties supérieure et inférieure de montage (12, 18) qui peuvent tourner l'une par rapport à l'autre, et en ce que la partie de montage inférieure comporte une partie externe (18B) qui porte contre la partie de montage supérieure pour transmettre les charges appliquées longitudinalement.

15. Prothèse selon l'une quelconque des revendications 12 à 14, caractérisée en ce que le composant de jambe (14) est un tube formant axe de jambe et en ce que une partie d'extrémité supérieure de l'élément de torsion (10) est fixée dans un manchon (12) monté intérieurement au tube de jambe (14), et une partie d'extrémité inférieure de l'élément de torsion (10) est fixée dans un logement (18) verrouillé à un ensemble de pied et/ou de cheville, le logement (18) comportant une surface de portée cylindrique (18A) qui vient porter contre une surface de portée interne correspondante dans le manchon (12), grâce à quoi le logement (18) peut tourner par rapport au manchon (12) autour de l'axe de la jambe, l'élément de torsion (10) définissant l'étendue de la rotation selon le couple appliqué au composant de jambe.

16. Prothèse selon l'une quelconque des revendications 12 à 15, caractérisée en ce que l'élément de torsion (10) est fabriqué en polyacétal, polyamide ou en un matériau plastique renforcé de fibres.

17. Prothèse selon l'une quelconque des revendications 12 à 16, caractérisée en ce que la longueur effective de l'élément de torsion (10) est réglable.

18. Prothèse selon l'une quelconque des revendications 12 à 17, caractérisée en ce que le composant principal de jambe (14) est un tube de jambe et l'autre partie d'extrémité de l'élément de torsion (10) est fixée à un élément s'étendant vers l'intérieur à l'intérieur du tube de jambe (14) en un endroit espacé de l'extrémité distale du tube de jambe (14), et dans laquelle l'autre partie d'extrémité de l'élément de torsion (10) est fixée à un logement (18) qui est reçu en rotation à l'intérieur de la partie d'extrémité distale du tube de jambe (14), une partie du logement (18) s'étendant longitudinalement à l'extérieur du tube de jambe (14) et étant reliée au pied.

19. Prothèse selon la revendication 18, caractérisée par un dispositif de fixation libérable (38) associé à l'autre extrémité de l'élément de torsion (10) et accessible à ou au voisinage d'une extrémité de ladite partie du logement (18) à l'extérieur du tube de jambe (14), le dispositif de fixation (38) retenant le logement (18) dans le manchon (12), et comprenant en outre des moyens libérables pour fixer ladite première partie d'extrémité de l'élément de torsion (10) à ladite partie qui s'étend vers l'intérieur du manchon (12).

20. Prothèse de membre inférieur dans laquelle le pied peut tourner par rapport à la jambe autour d'un axe s'étendant suivant la longueur de la prothèse, caractérisée par un composant de liaison tel que défini dans l'une quelconque des revendications 1 à 9.

21. Procédé de fabrication d'une prothèse de membre inférieur telle que définie dans l'une quelconque des revendications 12 à 20, caractérisé par l'étape consistant à choisir un élément de torsion (10) parmi divers éléments de torsion similaires présentant des rigidités différentes.

22. Procédé selon la revendication 21, caractérisé en ce que les éléments de torsion ainsi différents présentent des largeurs différentes, plus grande étant la largeur d'un élément plus grande étant sa rigidité rotationnelle autour de son axe longitudinal.
